# EUROPEAN PATENT SPECIFICATION

(11) **EP 3 700 559 B1**
(45) Date of publication and mention of the grant of the patent: **26.08.2026**
(21) Application number: 18811149.6
(22) Date of filing: 24.10.2018
(51) Int. Cl.: A61K 39/00, A61K 39/02

(54) **SINGLE STRAIN DYSENTERY VACCINE**
EINZELSTAMM IMPFUNG GEGEN SCHWEINEDYSENTERIE
VACCIN CONTRE LA DYSENTERIE PORCINE AVEC UNE SOUCHE UNIQUE

(30) Priority: 24.10.2017 EP 17382711
(43) Date of publication of application: 02.09.2020
(73) Proprietor: Aquilón Cyl S.L., 24006 León (ES); Universidad de León, 24071 León (ES)
(72) Inventor: RUBIO NISTAL, Pedro Miguel, 24007 Léon (ES); CARVAJAL URUEÑA, Ana María, 24007 Léon (ES); GARCÍA DÍEZ, Marta, 24007 Léon (ES)
(74) Representative: Hoffmann Eitle
(86) International application number: PCT/EP2018/079165
(87) International publication number: WO 2019/081583

(56) References cited:
- WO-A1-2014/207202
- WO-A1-2017/118581
- WO-A1-88/04555
- CARVAJAL A. ET AL: "Disenter�a Porcina: Tratamiento Y Control", AV. TECNOL. PORC, 1 May 2012 (2012-05-01), XP093342272
- EMEA Reflection paper on control of the active substance in the finished product for immunological veterinary medicinal products (IVMPs) XP093342052
- Directive 2001/82/EC XP093342275
- Wikipedia entry on inactivated vaccine, October 2019 XP093342055
- AVELINO ALVAREZ-ORDÓEZ ET AL: "Swine Dysentery: Aetiology, Pathogenicity, Determinants of Transmission and the Fight against the Disease", INTERNATIONAL JOURNAL OF ENVIRONMENTAL RESEARCH AND PUBLIC HEALTH, vol. 10, no. 5, 10 May 2013 (2013-05-10), pages 1927 - 1947, XP055430838, DOI: 10.3390/ijerph10051927

## Description

### Technical field

The present invention relates to a composition comprising a single strain of *Brachyspira hyodysenteriae* bacteria, particularly in the field of immunization against swine dysentery.

### Background art

Swine dysentery (SD), caused by colonic infection with the spirochaete *Brachyspira hyodysenteriae,* remains a major problem worldwide. It affects swine mainly during the fattening period. *Brachyspira hyodysenteriae* is a Gram-negative, oxygen-tolerant, anaerobic spirochete that colonizes the porcine large intestine to cause swine dysentery (SD). This condition is characterized by a severe mucohemorrhagic diarrhoea that primarily affects animals during the growing-finishing period and has been reported from all major pig-rearing countries (Hidalgo, A. et al., Journal of clinical microbiology (2010), 48(8):2859-2865).

SD is a widely distributed disease around the world, although studies regarding epidemiology are scarce and the reported prevalence significantly varies among them. Thus, *B. hyodysenteriae* reported prevalence ranges from 0 % to near 40 %. Variations in prevalence can be due to the use of different diagnostic methods or to differences among countries in housing, management, feeding regimes, etc. Moreover, whereas in many countries the prevalence may be concealed by the use of antimicrobials as feed additives, in others the ban of antibiotics as growth promoters may have resulted in an increase in SD prevalence (Alvarez-Ordóñez, A. et al., International Journal of Environmental Research and Public Health (2013), 10:1927-1947).

Carrier pigs play a main role in the epidemiology of swine dysentery and are considered the major source of transmission between herds. *B. hyodysenteriae* survives in the environment for long periods, especially in liquid faeces contained in pits and lagoons, where it may remain infective for up to 60 days. For instance, it can survive during several months in pig faeces at low temperatures. This spirochete also can naturally colonize mice, rheas, chickens, and mallards, and together with mechanical vectors or fomites, this increases the ways in which *B. hyodysenteriae* may be spread within and between herds (Hidalgo, A. et al., Journal of Clinical Microbiology (2010), 48(8):2859-2865).

The disease causes important direct financial losses, especially in intensive pig farms, derived from a decrease in food conversion efficiency, mortality, lengthening of the fattening period and also indirect losses, like an increase in veterinary expenses, medication, etc. The eradication of the disease through medication is quite difficult, since many clinically recovered animals keep shedding the organism for a long time while acting as carriers.

Treatment of SD involves the use of antibiotics. Pleuromutilins (tiamulin and valnemulin) have been used for this purpose in the European Union (EU). Tiamulin and valnemulin are semi-synthetic derivatives of the naturally occurring diterpene antibiotic pleuromutilin which show outstanding activity against anaerobic bacteria and are used exclusively in animals, largely in swine. Also macrolides (tylosin and, more recently, tylvalosin) and the closely related lincomycin (lincosamide) have been commonly included in SD therapeutic strategies. However, the emergence of B. *hyodysenteriae* strains with reduced susceptibility to one or more of these antibiotics and the presence of genetically diverse multiresistant isolates has been confirmed in several countries. This fact complicates treatment and control of SD and should alert veterinary surgeons and pig farmers for the need of a strategic approach to select antibiotics, which must only be used on strict indications following proper field and laboratory diagnosis in order to guarantee their long-term efficiency for SD treatment (Alvarez-Ordóñez, A. et al., International Journal of Environmental Research and Public Health (2013), 10:1927-1947).

The high costs of medication, together with the fact that on many occasions it is impossible to eradicate the infection completely, and the increasing worries about the presence of drug residues in both meat products and the environment, justifies the development of efficient immunoprophylactic methods to control SD (Diego, R. et al., Vaccine (1995), 13(7):663-667).

Large efforts have been made in order to develop vaccines to control SD since Joens and coauthors (Joens, L.A., et al., American Journal of Veterinary Research (1979), 40:1352-1354) reported that pigs which have recovered from acute SD are protected from disease when subsequently re-exposed to B. *hyodysenteriae,* indicating that the infection can induce a protective immune response (Alvarez-Ordóñez, A. et al., International Journal of Environmental Research and Public Health (2013), 10:1927-1947). However, attempts to develop vaccines to control SD have met with limited success. Hudson (Hudson, M.J. et al., British Veterinary Journal (1974), 130:37-40; Hudson, M.J. et al., Research in Veterinary Science (1976), 21:366-367) developed an attenuated live vaccine which was unable to protect against a subsequent challenge. Glock (Glock, R.D. et al., Proceedings of the 6th International Pig Veterinary Society Congress (1980), Copenhagen, Denmark, p. 521) reported some degree of protection upon challenge after six intravenous injections, at six-day intervals, of an inactivated vaccine. Attenuated or genetically modified live avirulent vaccines may show reduced colonization and cause less immune stimulation (Alvarez-Ordóñez, A. et al., International Journal of Environmental Research and Public Health (2013), 10:1927-1947).

An alternative approach is to generate subunit vaccines that might be delivered by the expression of recombinant *B. hyodysenteriae* proteins on a bacterial delivery vector. Efforts have been made to identify *B. hyodysenteriae* proteins for use in subunit vaccines, but vaccination with a recombinant 38 kDa flagellar protein failed to prevent colonization in experimentally infected pigs (Gabe et al., Infection and Immunity (1995), 63:142-148). On the other hand, vaccination with a recombinant 29.7 kDa outer membrane lipoprotein (Bhlp29.7) resulted in partial protection, with fewer animals developing disease than occurred in the control groups. The authors of this study concluded that vaccination also tended to delay the onset of faecal shedding of spirochaetes, but did not necessarily stop it from occurring (La, T. et al., Veterinary Microbiology (2004), 102:97-109). On a study conducted by Holden *et al*., the efficacy of vaccination with smpB (an outer membrane protein of *B. hyodysenteriae*) was evaluated. However, the response induced after protein vaccination offered only moderate protection against the disease (Holden, J. et al., Veterinary Microbiology (2008), 128:354-363). In most occasions recombinant vaccines tested have failed to provide enough protection in pigs (Alvarez-Ordóñez, A. et al., International Journal of Environmental Research and Public Health (2013), 10:1927-1947).

Vaccines consisting of whole cell bacterins induce serum antibody responses to *Brachyspira hyodysenteriae,* yet generally fail to protect pigs from disease. The use of *B. hyodysenteriae* bacterins prepared from whole cell lysates may even exacerbate disease upon infection (Waters, W.R. et al., Vaccine (2000), 18:711-719). Moreover, bacterin vaccines tend to be lipopolysaccharide serogroup-specific, which then requires the use of autogenous bacterins. Furthermore, *B. hyodysenteriae* bacterins are relatively difficult and costly to produce on large scale because of the fastidious growth requirements of the anaerobic spirochaete (La, T. et al., Veterinary Microbiology (2004), 102:97-109). In some countries, bacterin vaccines for SD are available commercially, and provide a degree of protection. However, as stated above, they tend to be lipooligosaccharide (LOS) serogroup specific, which then requires the use of autogenous or multivalent preparations (Hampson, D.J. et al., Diseases of Swine (2006), 10th Edition, Blackwell Publishing Professional, Ames, Iowa, U.S.A., pp. 687-688). Other references to SD vaccines in the art can be found in the following patent literature:
US 4748019: The authors found that an effective regime of vaccination comprises administering parenterally to pigs a priming dose of killed virulent or pathogenic *T. hyodysenteriae* effective to stimulate the immune response of the pig (strain "P18A", NCTC 11615) to a subsequent dose of a live avirulent or non-pathogenic strain of *T. hyodysenteriae* (strain "VSI", NCTC 11628) and at about the same time or thereafter administering this live strain orally.
US 5750118: The invention relates to a vaccine against SD comprising an effective quantity of inactivated and adjuvant-containing *T. hyodysenteriae* antigen (virulent or attenuated strain) for intradermal administration. The vaccine antigen is prepared from the strain No. 27164 ATCC, which is inactivated.
US 5281416: The invention relates to a method of vaccination of a pig against SD characterized by parenteral, preferably intramuscular administration to the pig of a live strain or of an oxygen-treated non-viable strain of *T. hyodysenteriae.* Representative strains which may be used are reference virulent strains ATCC 31287, ATCC 31212 and the reference avirulent strain ATCC 27164.
EP 3013363: The application relates to compositions and vaccines comprising combinations of different genetically diverse strains of B. *hyodysenteriae.* Further identified in the application are strains CNCM I-4720, CNCM I-4721 and CNCM I-4722.

However, the efficacy of these vaccines was found to be variable, or, in cases with different strains as ingredients of a vaccine, can be difficult to put into practice with regard to regulatory constraints. For example, according to European regulation, each active substance in a veterinary medicine composition must be both perfectly identified and quantified in the active substance. Therefore, if a composition comprises several strains, it can be necessary to develop tests that are able to both identify and quantify each strain individually. Such tests can be very difficult to develop from a technical point of view. Autogenous preparations (also known as "autovaccines", which may be defined as vaccines prepared from cultures of organisms isolated from the diseased animal's own tissues or secretions) have been used to further improve some of these vaccines. This approach, albeit efficient, is highly cost and time expensive and confers protection only for a single strain of B. *hyodysenteriae.* Moreover, the vaccination occurs sometime after the strain causing the disease has been identified, which can take several weeks (for instance, under standard procedures, the isolation process from the samples from the farm, initial culture and autovaccine production may take at least 6 weeks). This delay in time often causes the propagation of the bacteria in other animals from the herd, or in extreme circumstances, even to other pig farms. It also provokes serious economic losses and it is itself an expensive procedure to be applied on routine basis. SD thus remains an important endemic infectious disease in many pig rearing countries. There is a huge necessity of an effective and economically affordable single strain based vaccine for SD.

### Summary of the invention

Swine Dysentery (SD) is a severe mucohaemorhagic enteric disease of pigs caused by *Brachyspira hyodysenteriae,* which has a large impact on pig production and causes important losses due to mortality and sub-optimal performance. Considering the emergence of multi-resistant strains and the concern that drug residues may be present in meat products or the environment, efficient immunoprophylactic methods to control SD are urgently needed. However, the available single-strain vaccines fail to confer a satisfactory degree of protection against infection and, even if they confer a certain degree of protection, they do not provide adequate cross-protective immunity against strains of different MLVA types, clonal complexes and/or serogroups. Moreover, the fabrication and commercialization of autovaccines present many inconveniences. Finally, it should be noted that compositions that comprise a combination of multiple strains can suffer from technical and regulatory constraints. Accordingly, there is a necessity of vaccines against SD which are based on a single strain and which nevertheless confer protection against different strains, namely an effective and single-strain based SD vaccine.

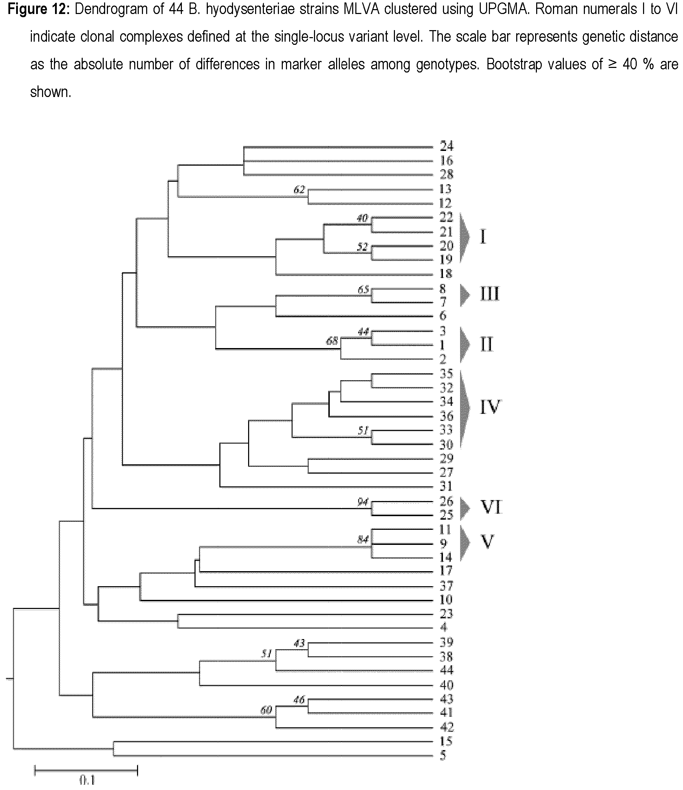

The inventors have developed an effective vaccine against SD based on a single strain that provides protection against SD, and demonstrated the effects with a challenge with the strain B204, a reference strain for the evaluation of vaccines against SD. This strain is deposited in the American Type Culture Collection (ATCC) as ATCC 31212 and publically available. The strain is herein also referred to as the challenge strain. This strain B204 is not particularly closely related to the strain of the present invention (see Figure 12: CNCM I-4720 is MLVA type 3, B-204 is MLVA type 23). This effect is therefore highly surprising, as it is in conflict with the autovaccine theory as well as the theory that multiple diverse strains would be necessary to grant a broader protection against different *Brachyspira hyodysenteriae* strains. This protection against a distant strain shows that the protection granted by the vaccine of the present invention is not limited to closely related strains, as e.g. autovaccines, but confers a broad protection against different *Brachyspira hyodysenteriae* strains. Such a protection, which is not limited to strains that are closely related to the strain used as a vaccine, but instead confers protection also against distant strains of e.g. different clonal complexes, is herein referred to as a universal protection, and the vaccine is accordingly referred to as a universal vaccine.

In a first aspect, the present invention provides a composition as claimed for the claimed use comprising bacteria from a single strain of *Brachyspira hyodysenteriae,* wherein said strain is the strain that has been deposited by the applicant at the *Collection Nationale de Cultures de Microorganismes* (CNCM), Institut Pasteur, 28, Rue du Docteur Roux, 75724 Paris Cédex 15, France, on March 14, 2013, with registration number CNCM I-4720 (hereinafter also referred to as strain CNCM I-4720 or strain H57 or the strain of the present invention).

The present invention is related to the composition of the invention for its use as a vaccine against swine dysentery caused by *Brachyspira hyodysenteriae.* Moreover, disclosed is a method for producing the composition of the invention, at a concentration of at least between 10⁸ and 10⁹ bacteria/mL.

The present invention can in particular be used for the prevention and/or treatment of diarrhea, for example mucous and/or bloody diarrhea, and swine dysentery, using any of the compositions and/or vaccines described herein.

The invention is set out in the claims. Any references in the description to methods of treatment refer to the compounds, pharmaceutical compositions and medicaments of the present invention for use in a method for treatment of the human (or animal) body by therapy.

### Brief description of the figures

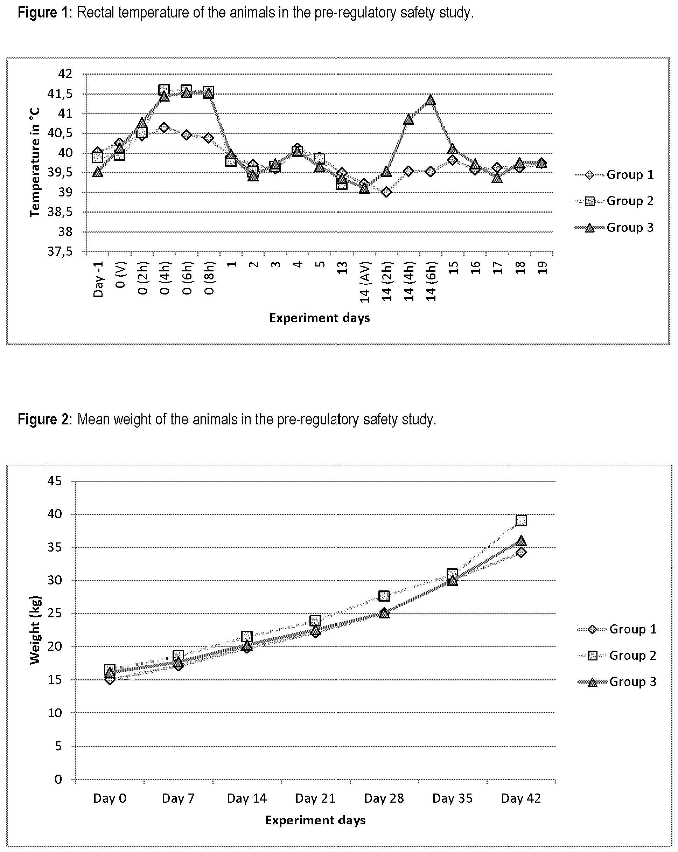

**Figure 1****:** Rectal temperature of the animals in the pre-regulatory safety study. Grey: animals treated with a physiological solution (group 1), light grey: animals that received the vaccine of the present invention once at day 0 (group 2), dark grey: animals that received the vaccine of the present invention twice, once at day 0 and once at day 14 (group 3). Each group consisted of 8 animals.
**Figure 2****:** Mean weight of the animals in the pre-regulatory safety study. Grey: animals treated with a physiological solution (group 1), light grey: animals that received the vaccine of the present invention once at day 0 (group 2), dark grey: animals that received the vaccine of the present invention twice, once at day 0 and once at day 14 (group 3). Each group consisted of 8 animals.

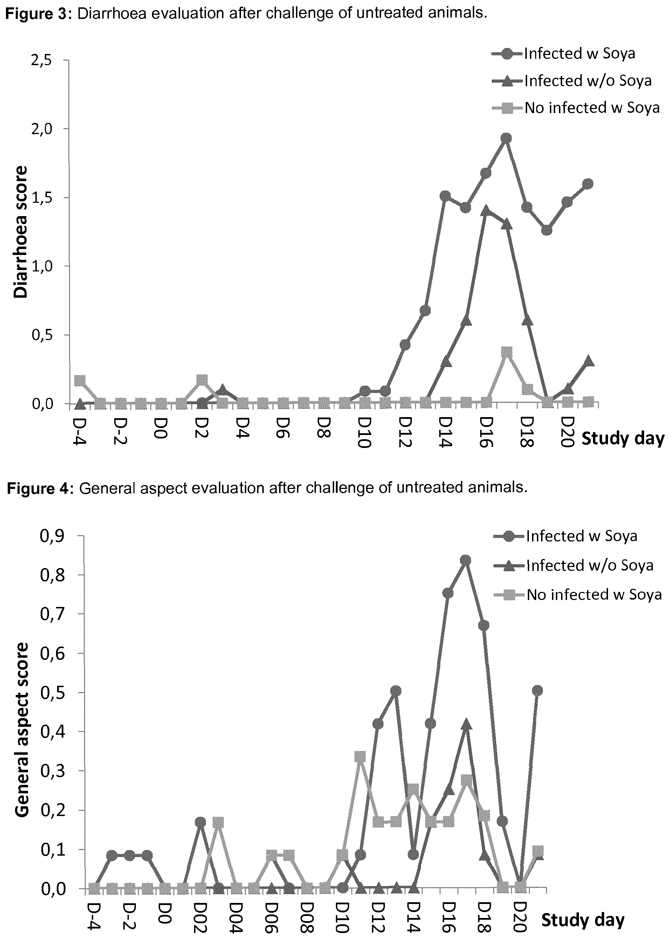

**Figure 3****:** Diarrhoea evaluation after challenge of untreated animals. Dark grey: Animals challenged with the challenge strain and fed with hyperproteic (soy rich) feed. Light grey: Animals challenged with the challenge strain and fed with normal feed. Medium grey: Unchallenged animals fed with hyperproteic (soy rich) feed.
**Figure 4****:** General aspect evaluation after challenge of untreated animals. The following parameters were evaluated: dyspnoea, nasal discharge, cough, conjunctivitis, sneezing, paralysis, discoordination, lethargy, vomiting, external aspect. 0: Not altered, 1: Mild/moderate alteration, 2: Severe alteration. Dark grey: Animals challenged with the challenge strain and fed with hyperproteic (soy rich) feed. Light grey: Animals challenged with the challenge strain and fed with normal feed. Medium grey: Unchallenged animals fed with hyperproteic (soy rich) feed.

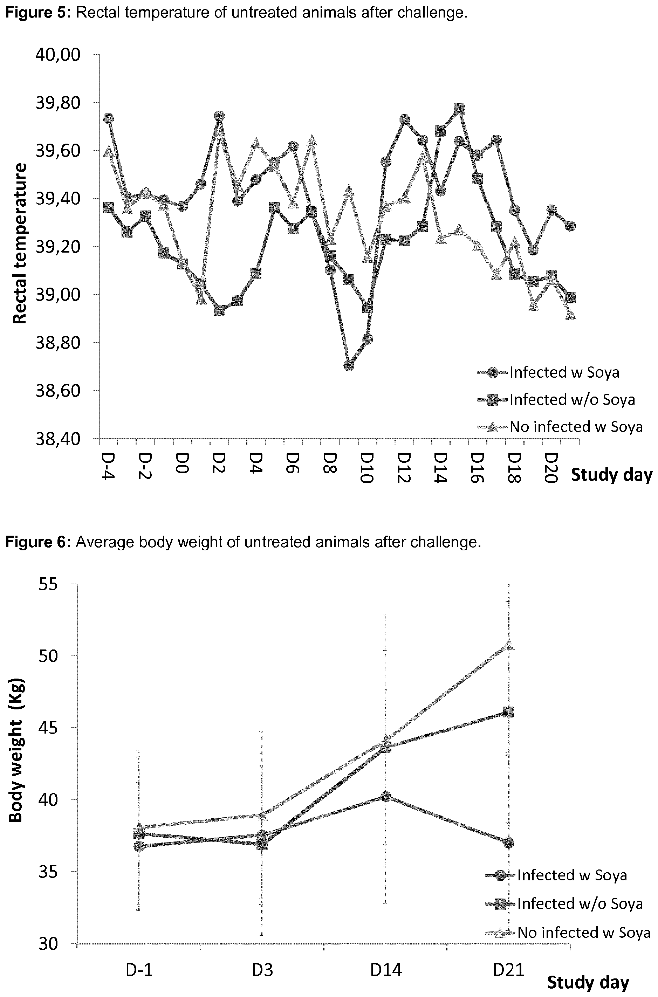

**Figure 5****:** Rectal temperature of untreated animals after challenge. Dark grey: Animals challenged with the challenge strain and fed with hyperproteic (soy rich) feed. Light grey: Animals challenged with the challenge strain and fed with normal feed. Medium grey: Unchallenged animals fed with hyperproteic (soy rich) feed.
**Figure 6****:** Average body weight of untreated animals after challenge. Dark grey: Animals challenged with the challenge strain and fed with hyperproteic (soy rich) feed. Light grey: Animals challenged with the challenge strain and fed with normal feed. Medium grey: Unchallenged animals fed with hyperproteic (soy rich) feed.

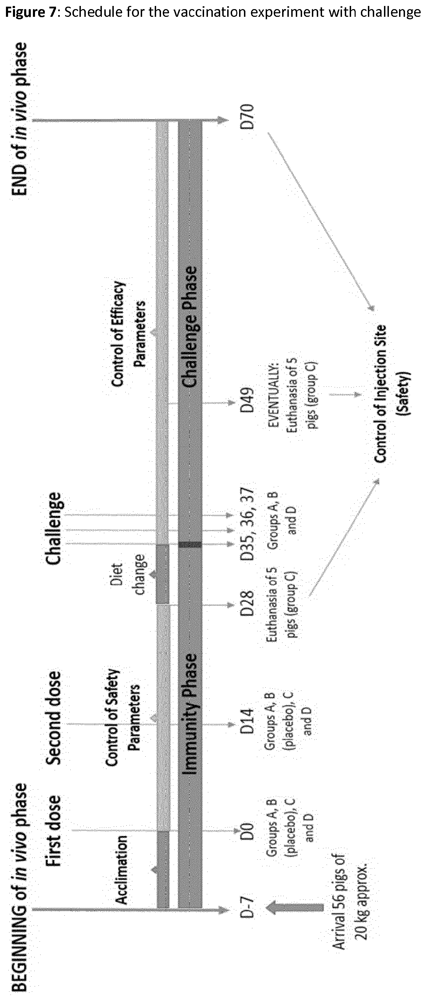

**Figure 7****:** Schedule for the vaccination experiment with challenge. All animals were sacrificed at day 71 of the trial.

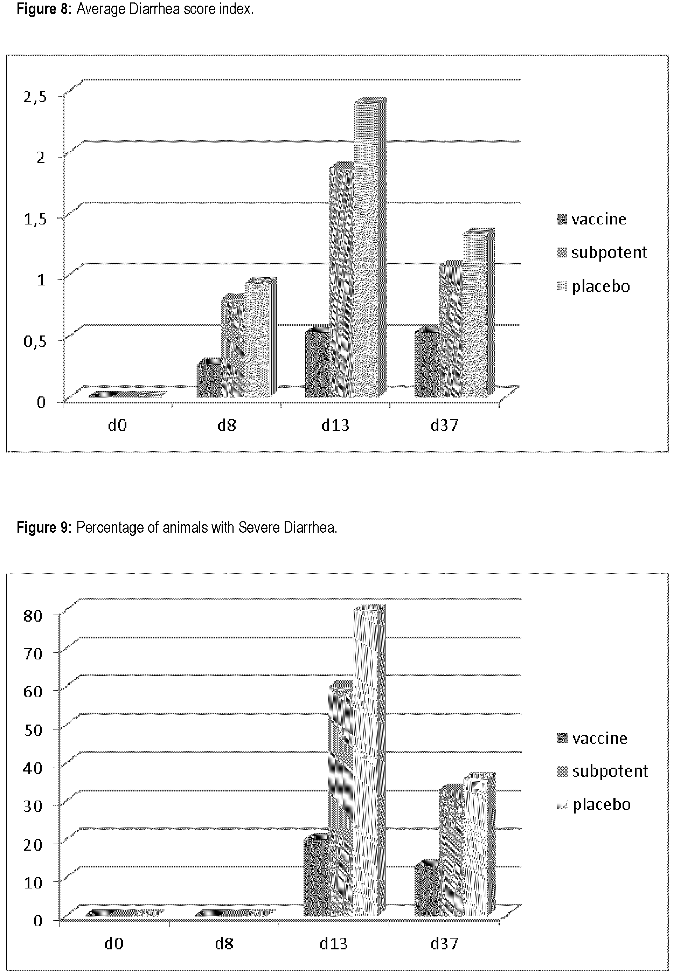

**Figure 8****.** Average Diarrhea score index. Stool quality was measured using a three score criteria (0, 2, 4) after the 3rd day of the challenge (d0) and at days 8, 13 and 37 (d8, d13, d37). Stool scores were 0 for normal or loose consistency, 2 for liquid diarrhea and 4 for mucous or bloody liquid diarrhea, considered severe diarrhea. Dark grey: vaccinated animals; grey: animals vaccinated with subpotent vaccine; light grey: animals treated with placebo.
**Figure 9****.** Percentage of animals with Severe Diarrhea. Stool quality was measured after the 3rd day of the challenge (d0) and at days 8, 13 and 37 (d8, d13, d37). Animals showing mucous or bloody liquid diarrhea were considered to have severe diarrhea. Dark grey: vaccinated animals; grey: animals vaccinated with subpotent vaccine; light grey: animals treated with placebo.

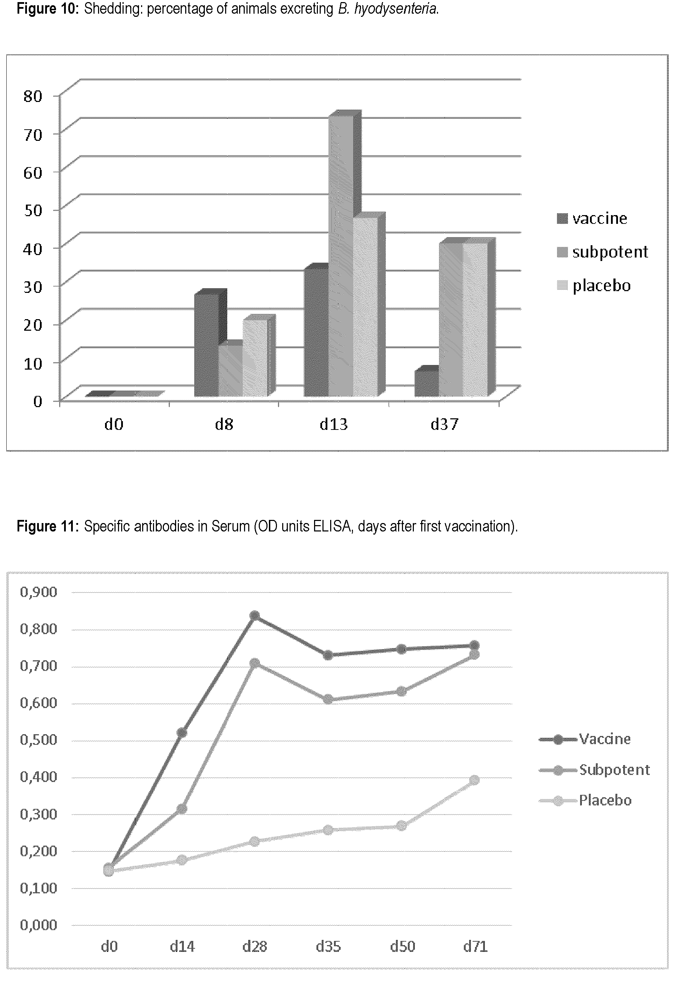

**Figure 10****.** Shedding: percentage of animals excreting *B. hyodysenteria.* Presence or absence of Brachyspira in individual stools was measured by PCR after the 3rd day of the challenge (d0) and at days 8, 13 and 37 (d8, d13, d37). Dark grey: vaccinated animals; grey: animals vaccinated with subpotent vaccine; light grey: animals treated with placebo.
**Figure 11****.** Specific antibodies in Serum after the first vaccination (d0) and subsequent days. The animals received the second shot 14 days after the first vaccination (d14). Dark grey: vaccinated animals; grey: animals vaccinated with subpotent vaccine; light grey: animals treated with placebo.
**Figure 12****.** Dendrogram of 44 B. hyodysenteriae MLVA clustered using UPGMA. Roman numerals I to VI indicate clonal complexes defined at the single-locus variant level. The scale bar represents genetic distance as the absolute number of differences in marker alleles among genotypes. Bootstrap values of ≥ 40 % are shown. CNCM I-4720 is MLVA type 3, B-204 is MLVA type 23.

### Detailed description of the invention

Throughout the description and claims the word "comprise" and variations of the word typically is not limiting and thus does not exclude other features, which may be for example technical features, additives, components, or steps. However, whenever the word "comprise" is used herein, this also includes a special embodiment in which this word is understood as limiting; in this particular embodiment the word "comprise" has the meaning of the term "consist of".

### Methods of the invention

In the present invention, statements relating to strains, compositions and/or vaccines have to be understood to also refer to uses in treatment using said strains, compositions and/or vaccines.

For example, the present invention refers to uses of the claimed compositions in methods of prevention and/or treatment of diarrhea, for example mucous and/or bloody diarrhea, and/or swine dysentery, using any of the strains, compositions and/or vaccines described herein.

In the present invention, statements relating to strains, compositions and/or vaccines have to be understood to also refer to the use of said strains, compositions and/or vaccines for the manufacture of a medicament. For example, the present invention refers to the use of strains, compositions and/or vaccines for the manufacture of a medicament. Preferably, said medicaments are for the prevention and/or treatment of diarrhea, for example mucous and/or bloody diarrhea, and/or swine dysentery.

The terms "treatment" or "therapy" encompass both prophylactic and curative methods of treating disease, since both are directed to the maintenance or restoration of health. Irrespective of the origin of pain, discomfort or incapacity, its relief, by the administration of an appropriate agent, is to be construed as therapy or therapeutic use in the context of the present application.

The composition and vaccine of the invention may thus be used in a method of therapeutic treatment (after the clinical manifestation of the disease (diarrhoea) and/or prophylactic treatment (before the clinical manifestation of the disease (e.g., diarrhoea)).

### Composition of the invention

The present invention relates to a composition as claimed for the claimed use comprising bacteria of strain CNCM I-4720. As demonstrated herein, such a composition can be used as an effective vaccine for the prevention and/or treatment of SD caused by distant strains.

A relatively high diversity among B. *hyodysenteriae* isolates has been classically described. The ability to understand the epidemiology of SD and to progress to its control depends on the availability of reliable strain typing methods to characterize the isolates. Based on the analysis of semi-purified lipopolysaccharides (LPS), four different serotypes were identified by Baum & Joens (Baum, D.H. et al., Infection and immunity (1979), 25:792-796), although further studies finally differentiated a total of 11 serogroups that included several serotypes (Hampson, D.J. et al., Epidemiology and Infection (1989), 102:75-84; Hampson, D.J. et al., Epidemiology and Infection (1990), 105:79-85; Hampson, D.J. et al., Swine dysentery. In: Intestinal Spirochaetes in Domestic Animals and Humans, pp. 175-209, edited by D. J. Hampson & T. B. Stanton. Wallingford: CAB International, 1997).

Differences in the geographical distribution of B. *hyodysenteriae* were demonstrated soon after. Reference strains from USA were classified within serotypes 1 and 2 while a higher variability regarding serotype classification was described for isolates from Europe and Australia (Harris et al., Swine Dysentery. In: Straw, B.E., D'Allaire, S.D., Mengeling, W.D. & Taylor, D.J. (Eds.) Disease of Swine. Iowa State University Press (1999), Ames lowa USA, pp. 579-600). However, there is almost no recent information regarding serotype distribution of *B. hyodysenteriae* isolates. As a consequence of cross reactions, the techniques required to determine the serotype are slow and cumbersome to perform and give inconclusive results in a very high number of the isolates. For that reason, these techniques have been replaced by several molecular methods.

Different typing tools have been developed to discriminate between B. *hyodysenteriae* field isolates and provide a better understanding of the molecular epidemiology of the pathogen. Among them, a useful tool for strain typing of pathogenic microorganisms that has been introduced during the last few years is the multi-locus variable-number tandem-repeat analysis or MLVA. It has been developed as an important epidemiologic tool for strain typing of pathogenic microorganisms. MLVA is based on the PCR amplification of a number of well-selected and characterized loci that contain short repeat sequences (multiple loci of minisatellites called variable numbers of tandem repeats (VNTRs)). This sort of minisatellite consists of unique direct head-to-tail DNA repeats which can be found in all bacterial genomes and can be used to define specific isolates of bacterial species. In addition, VNTRs have been used to infer the bacterial population structure and phylogeny of diverse bacteria species. Within each repeat sequence locus the number of repeat copies can vary between different strains. By measuring the size of each PCR amplified loci, the number of repeat units can be deduced. Hidalgo and colleagues developed and tested a multiple-locus variable-number tandem-repeat analysis (MLVA) method that could be used in basic veterinary diagnostic microbiology laboratories equipped with PCR technology or in more advanced laboratories with access to capillary electrophoresis. Based on eight loci, and when performed on isolates from different farms in different countries, as well as type and reference strains, the developed MLVA technique was highly discriminatory (Hunter and Gaston discriminatory index, 0.938 [95% confidence interval, 0.9175 to 0.9584]) while retaining a high phylogenetic value. Using the technique, the species was shown to be diverse (44 MLVA types from 172 isolates and strains), although isolates were stable in herds over time. The population structure appeared to be clonal. The finding of *B. hyodysenteriae* MLVA type 3 in piggeries in three European countries, as well as other, related, strains in different countries, suggests that spreading of the pathogen via carrier pigs is likely. MLVA overcomes drawbacks associated with previous typing techniques for *B. hyodysenteriae* and is a powerful method for epidemiologic and population structure studies on this important pathogenic spirochete (Hidalgo, A. et al., Journal of Clinical Microbiology (2010), 48(8):2859-2865).

The inventors and their collaborators have applied this method on an international collection of *B. hyodysenteriae* isolates, including 115 Spanish field isolates as well as reference strains and isolates from Australia, Canada, E.E.U.U., UK and The Netherlands.

MLVA analysis reveals that Spanish field isolates of *B. hyodysenteriae* are heterogeneous and that the population has a clonal structure. A total number of 15 MLVA types were identified among Spanish isolates. Moreover, isolates with the same MLVA type were identified in Spain, UK and The Netherlands. On the other hand, it was concluded that isolates from Australia or EEUU have no common MLVA with Spanish isolates.

By grouping MLVA types at the single-locus variant level, a total number of six clonal complexes (I to VI) were established (Figure 12).

The common ancestor within each clonal complex was predicted using the goeBUST algorithm available at http://goeburst.phyloviz.net/#Software, a global implementation of the eBURST algorithm. For more details see publications Feil *et al.,* 2004 and Francisco *et al.,* 2009, free at http://goeburst.phyloviz.net/#Publications.

The present invention uses the strain with registration number CNCM I-4720 deposited within the *Collection Nationale de Cultures de Microorganismes* (CNCM), Institut Pasteur, on March 14, 2013. The strain with registration number CNCM I-4720 belongs to clonal complex II, more specifically to the ancestral type of clonal complex II. The present inventors surprisingly found that a vaccine based on this strain is able to confer protection against a reference strain of another MLVA type which does not even belong to clonal complex II. Said single-strain vaccine therefore confers a protection not only against strains from a single MLVA type, but also against more distant strains that are of a different MLVA type or do not even belong to the same clonal complex.

In some of the embodiments, the compositions disclosed herein comprise no other bacterial strain than strain CNCM I-4720 ("single strain composition"). Preferably, the bacteria comprised in the composition are in an inactivated form. The single strain composition of the present invention is used in a method of preventing and/or treating swine dysentery, preferably reducing the occurrence of mucous and/or bloody diarrhea, and/or delaying the appearance of clinical signs, and/or reducing the overall severity of diarrhea and/or severe diarrhea, and/or preventing and/or reducing the time of shedding of *Brachyspira hyodysenteriae,* and/or of helping to develop diarrhea only at later stages and/or resolving diarrhea sooner when compared to non-vaccinated animals and/or animals vaccinated with a low-dose vaccine. The single strain composition of the present invention is used in the prevention and/or treatment of swine dysentery. The single strain composition of the present invention is effective in the prevention and/or treatment of swine dysentery caused by *B. hyodysenteriae* strains that are heterologous (compared to strain CNCM I-4720). The single strain composition of the present invention is effective in the prevention and/or treatment of swine dysentery caused by *B. hyodysenteriae* strains that are of another clonal complex and/or another MLVA type optionally of a different serogroup than the strain CNCM I-4720. In some of the embodiments, the single strain composition of the present invention is effective in the prevention and/or treatment of swine dysentery caused by *B. hyodysenteriae* strains that are of the same clonal complex and/or MLVA type and/or serogroup than the strain CNCM I-4720. In some of the embodiments, the single strain composition of the present invention provides protection against different strains of different serogroups. In some of the embodiments, the single strain composition of the present invention represents an effective and universal single-strain based SD vaccine.

In some of the embodiments, the term "*single strain composition*" refers to a composition wherein the bacterial strain CNCM I-4720, preferably in an inactivated form, is the only immunogenic component derived from a bacterium, and preferably the only immunogenic component. "Derived from a bacterium" means any protein, peptide, DNA, RNA etc. that originates from a bacterium and/or leads to the same or similar specific immunogenic response as a protein or peptide or DNA or RNA from a bacterium, e.g. encodes for a protein or peptide that has the same or a similar immunogenic response. In yet another embodiment, "single strain composition" means that the only immunogenic ingredient is the strain referred to (strain CNCM I-4720), preferably in an inactivated form.

Moreover, the bacteria comprising the composition of the present invention are inactivated, i.e. they may be chemically or physically inactivated. The inactivation comprises killing the bacteria with chemicals, heat, and/or radiation. The bacteria of the composition can be inactivated by any inactivation procedure known in the art. Preferably, the bacteria of the composition of the invention are inactivated by treating the bacteria with formaldehyde. Most preferably, the formaldehyde is injected in to the bacteria culture at 0,5% and it is then incubated overnight (18 hours, approx.) at 37 °C with light agitation.

Generally, the use of inactivated strains as vaccines may have several advantages compared to live vaccines. For example, vaccines based on inactivated strains may be easier to handle and store, as they are less sensitive to heat or light. Inactivated strains are safer in the sense that they cannot regain virulence and cause an infection.

According to the present invention, the bacteria of the composition, which are inactivated, may be present in a concentration of at least between 10⁷ and 10¹² bacteria/mL, preferably in a concentration of at least 10⁷, or 10⁸, or 5·10⁸, or 10⁹, or 10¹⁰, or 10¹¹ or 10¹² bacteria/mL, preferably in a concentration of between 10⁸ and 10¹⁰ bacteria/mL, more preferably in a concentration of between 10⁸ and 10⁹ bacteria/mL, even more preferably in a concentration of 5·10⁸ bacteria/mL. In accordance with the invention, these numbers refer to bacteria from a single strain of B. *hyodysenteriae.*

The concentration of bacteria in the composition can be calculated using any method known in the art. For example, Neubauer chamber counting can be used to estimate the number of bacteria present in the composition of the invention. This method can also be applied to inactivated bacteria.

The composition of the present invention may further comprise an adjuvant. An adjuvant is a component that potentiates the immune response to an antigen and/or modulates it towards the desired immune responses. It may be an inorganic or organic chemical, macromolecule or whole cells of certain killed bacteria which enhances the immune response to given antigen. In the context of the present invention, the adjuvant that may be present in the composition of the invention can be any suitable adjuvant which e.g. enhances, accelerates and prolongs the specific immune response as known in the current art.

Adjuvants may include for instance:
- Mineral salts, e.g., aluminium hydroxide and aluminium or calcium phosphate gels.
- Oil emulsions and surfactant based formulations, e.g., MF59 (microfluidised detergent stabilised oil-in-water emulsion), QS21 (purified saponin), AS02 [SBAS2] (oil-in-water emulsion + MPL + QS-21), Montanide^{™} ISA-51, ISA-720, IMS (stabilised water-in-oil emulsion).
- Particulate adjuvants, e.g., virosomes (unilamellar liposomal vehicles incorporating influenza haemagglutinin), AS04 ([SBAS4] Al salt with MPL), ISCOMS (structured complex of saponins and lipids), polylactide co-glycolide (PLG).
- Microbial derivatives (natural and synthetic), e.g., monophosphoryl lipid A (MPL), Detox (MPL + M. Phlei cell wall skeleton), AGP [RC-529] (synthetic acylated monosaccharide), DC_Chol (lipoidal immunostimulators able to self organise into liposomes), OM-174 (lipid A derivative), CpG motifs (synthetic oligonucleotides containing immunostimulatory CpG motifs), modified LT and CT (genetically modified bacterial toxins to provide non-toxic adjuvant effects).
- Endogenous human immunomodulators, e.g., hGM-CSF or hIL-12 (cytokines that can be administered either as protein or plasmid encoded), Immudaptin (C3d tandem array)
- Inert vehicles, such as gold particles towards the desired response to vaccine antigens.

The most preferred adjuvants are aluminum salts (aluminum hydroxide or aluminum phosphate) and mineral oils. When inoculated they produce a small granuloma that allows the delayed liberation of the antigen (long lasting antigenic stimulation) and the attraction of antigen-presenting cells. This increases the immune response. For example, the adjuvant may be HAVLOGEN^{™} or Montanide^{™}. Most preferably, the adjuvant may be a commercial oil adjuvant such as Montanide^{™} IMS 251 C VG (SEPPIC).

The adjuvant is preferably present in the final composition in a concentration in the final formula of 5 to 50 % vol/vol respect to final injection volume, preferably 5 %, 10 %, 20 %, 25 %, 30 %, 40 %, 50 % or more (vol/vol, i.e. volume with respect to final injection volume). More preferably, the concentration of adjuvant in the final formula is 20 % vol/vol (i.e. volume with respect to final injection volume).

The composition of the invention may additionally comprise other components. For example, the composition may comprise antiseptic and/or antifungal agents. For example, the composition may further comprise Thimerosal (Sigma), also known as Thiomersal. In some embodiments, Thimerosal is comprised in an amount of 0.005 to 1 g per 100 ml, preferably in an amount of 0.5, or 0.3 or 0.1, or 0.05, or 0.03, or 0.02, or 0.01 or 0,005 g per 100 ml. In some embodiments, thimerosal is comprised in an amount of 0.01 g per 100 ml. In a particularly preferred embodiment, the composition does not comprise Thimerosal. In an even more preferred embodiment, the composition does not comprise any mercurial derivatives. In a most preferred embodiment, the composition does not comprise any heavy metals and/or preserving agents. Further, the composition of the invention may also comprise buffer solutions such as salts. Preferably, the composition of the invention may comprise a buffer in a concentration of 0.01 to 0.5 M, preferably in a concentration of 0.5M, or 0.4M, or 0.3M, or 0.2M, or 0.1M, or 0,08M or 0.05M, or 0.01M. The buffer may be any suitable buffer described in the art. For example, the buffer may be phosphate buffered saline (PBS) or sodium acetate. Preferably, the buffer is sodium acetate 0.1M. The composition of the invention may also comprise pharmaceutically acceptable carriers.

As used herein, the expression "pharmaceutically acceptable carrier" means a non-toxic solvent, dispersant, excipient, adjuvant, or other material which is mixed with the bacteria of the present invention in order to permit the formation of a pharmaceutical composition, i.e., a dosage form capable of administration to the animals. For example, water could be considered as a pharmaceutically acceptable carrier suitable for the present invention. For example, a buffer/saline solution could be considered as a pharmaceutically acceptable carrier suitable for the present invention.

The composition of the invention may also comprise water and/or salts and/or buffers.

### Vaccine of the invention

The composition of the present invention may be preferably used as a vaccine. A vaccine is a biological preparation that improves immunity to a particular disease. According to the present invention, the vaccine is a vaccine against swine dysentery (SD) caused by *Brachyspira hyodysenteriae.*

The composition of the invention for use as a vaccine (from now on, the vaccine of the invention) is a single strain swine dysentery (SD) vaccine, wherein the swine dysentery is caused by *Brachyspira hyodysenteriae.* In one embodiment, the term "*single strain vaccine*" refers to a vaccine which comprises no other bacterial strain than strain CNCM I-4720, preferably in an inactivated form. In another embodiment, the term "*single strain vaccine*" refers to a vaccine wherein the bacterial strain CNCM I-4720, in an inactivated form, is the only immunogenic component derived from a bacterium, and preferably the only immunogenic component. "Derived from a bacterium" means any protein, peptide, DNA, RNA etc. that originates from a bacterium or leads to the same or similar specific immunogenic response as a protein or peptide from a bacterium, e.g. encodes for a protein or peptide that has the same or a similar immunogenic response.

The vaccine of the invention may be suitable for administration to swine in a particular geographical region of interest. As described above, the region of interest is not particularly limited, and may comprise one or more countries. For example, the region of interest can be Europe. Preferably, the region of interest can be Spain, more preferably Iberian Peninsula Spanish territory, in particular Castilla y León, Andalucia and/or Extremadura. Other preferred regions of interest are Italy, The Netherlands, United Kingdom, Australia, Canada and/or United States.

The vaccine of the invention may be administered before the infection, and/or shortly after it. For example, the vaccine of the invention may be administered 1 to 20 days after the outbreak of the disease, preferably 1, or 2, or 3, or 4, or 5, or 6, or 7, or 8, or 9, or 10, or 15, or 20 days after the outbreak of the infection. The vaccine may also be administered 1-4 weeks after the outbreak of the disease, preferably 1, 2, 3 or 4 weeks after the outbreak of the infection.

The vaccine of the invention may be administered by parenteral administration and/or oral administration. Preferably, the vaccine of the invention is administered by parenteral administration, more preferably by subcutaneous and/or intramuscular and/or intradermal administration, and even more preferably by intramuscular administration. For example, the vaccine of the invention may be injected intramuscularly into the neck muscles of swine.

The administered dosage of the vaccine of the invention may range from 1 mL to 5 mL. For example, one dosage of the vaccine of the invention may be 1 mL. For example, one dosage of the vaccine of the invention may be 2 mL.

The administered dosage of the single-strain vaccine of the invention may comprise between 10⁷ and 10¹² bacteria/dose, preferably between 10⁸ and 10¹⁰ bacteria/dose, more preferably 10⁹ bacteria/dose. The administered dosage of the vaccine of the invention may comprise between 10⁸ and 10⁹ bacteria/mL. The administered dosage of the vaccine of the invention may comprise 10⁹ bacteria/mL. Preferably, the administered dosage of the vaccine of the invention may comprise 5·10⁸ bacteria/mL in a 2 mL/dose.

Accordingly, the preferred total number of bacteria per dose which may be administrated to swine is 10⁹ bacteria.

The single-strain vaccine of the present invention is an injectable vaccine, preferably an intramuscularly injectable vaccine.

The single-strain vaccine of the present invention preferably elicits an immune response based on antibodies. In a further embodiment, the single-strain vaccine of the present invention elicits a dose-dependent immune response.

According to the present invention, the vaccine of the present invention may be used to treat or prevent diarrhea. In some embodiments, the vaccine may be used to delay the appearance of clinical signs and/or to reduce the overall severity of diarrhea and/or severe diarrhea. In some embodiments, the vaccine of the present invention may also be used to prevent and/or reduce the time of shedding of *Brachyspira hyodysenteriae.* In some embodiments, the vaccine of the present invention may also be used to help develop diarrhea only at later stages and/or to resolve diarrhea sooner when compared to non-vaccinated animals and/or animals vaccinated with a low-dose vaccine.

### Vaccination protocol

According to the present invention, the vaccine of the invention may be preferably administered to the swine after weaning, most preferably two weeks after weaning. For example, the swine may be vaccinated since the fourth week of life. According to the present invention, the swine may be vaccinated twice (revaccinated). The swine are preferably revaccinated two weeks after the first vaccination. For example, the swine may be vaccinated two weeks after weaning, e.g. at the age of four weeks. Then, the second vaccine (revaccination) may take place at the age of six weeks (i.e. two weeks after the first vaccine was administered). For example, the swine may be vaccinated two weeks after weaning, e.g. at the age of five weeks. Then, the second vaccine (revaccination) may take place at the age of seven weeks. For example, the swine may be vaccinated two weeks after weaning, e.g. at the age of six weeks. Then, the second vaccine (revaccination) may take place at the age of eight weeks. Vaccination for the first time at the age of six weeks is preferred.

Weaning can occur at 21 days of age (La, T. et al., Veterinary Microbiology (2004), 102:97-109). Weaning can also occur at 15 days of age, or at any other age, depending on the heard.

### Challenge

According to the present invention, the efficiency of the vaccine can be evaluated by challenging animals that were treated with a vaccine of the present invention in comparison to untreated animals or animals that have been treated with a composition with a reduced dose of bacteria compared to the vaccine of the present invention.

According to the present invention, animals can be challenged with a *Brachyspira hyodysenteriae* strain. This strain can be the same or a different strain than the strain CNCM I-4720. Preferably, the strain is a different strain, and most preferably the strain is the strain B204. B204 is a well-characterized strain and its use as a challenge strain in the regulatory studies was supported by the European Medicines Agency (EMA) after a specific question addressing this issue in an official scientific advice. This strain is deposited in the American Type Culture Collection (ATCC) as ATCC 31212 and publically available. It can be requested for research centers or other accredited facilities. The strain used for the challenge is hereinafter referred to as the "challenge strain".

According to the present invention, the challenge may be performed by inoculating the animals with a composition comprising live bacteria of the challenge strain, preferably strain B-204. Preferably, the inoculum is administered orally. According to the present invention, the inoculum may for example comprise between 10⁴ and 10¹⁰ bacteria of the challenge strain per dose, more preferably between 10⁵ and 10⁹ bacteria of the challenge strain per dose, even more preferably between 10⁶ and 10⁷ bacteria of the challenge strain per dose, and most preferably 5 x 10⁶ bacteria of the challenge strain per dose. According to the present invention, the inoculum may for example have a concentration of between 10⁴ and 10¹⁰ bacteria/mL of the challenge strain per dose, more preferably between 10⁵ and 10⁹ bacteria/mL of the challenge strain per dose, even more preferably between 10⁶ and 10⁷ bacteria/mL of the challenge strain per dose, and most preferably 5 x 10⁶ bacteria/mL of the challenge strain per dose, each inoculation dose corresponding to 50mL, wherein three consecutive doses are administered to the animals. The inoculum may be solid or liquid. The inoculum is preferably liquid, and most preferably is a bacterial suspension. The inoculum may for example be administered once, once a week, once every three days, once every two days, once a day, twice a day or several times a day, most preferably once a day. The inoculum may for example be administered over the course of two weeks, over the course of a week, over four days, over three days, over two days or over one day. Most preferably, the inoculum is administered once a day over the course of three days.

To do this, sterile 60 ml syringes may be used. To do this, the plunger may be removed from the syringe and the nozzle may be covered with one finger. Then, the syringe body may be filled with the inoculum. The plunger may then be replaced and the remaining air may be removed. After doing that, the mixture may be introduced directly into the mouth of the animal by expelling the contents of the syringe into the mouth, and checking that the animal takes all the mixture.

According to the present invention, the bacteria to be used for the challenge can for example be obtained by the following steps:

### Step 1: growing the bacteria on a plate

The challenge strain, preferably strain B-204, can be seeded in 9 blood agar (BA) plates with a 10 µl sowing loop and can then be incubated at 38.5 °C in anaerobic conditions until hemolysis-growth can be observed (1-2 days). This is called pass 1 (P1). When the strain grows, the absence of contamination can be checked, e.g. under a microscope. If the strain is pure, growth can be collected with a loop, taking agar fragments in depth at the hemolysis edges (which have a better quality of the culture), and can be deposited on a new BA plate. These fragments can be homogenized and spread in zigzag movements along the plate with the loop. Each of the 9 initial plates can then be seeded into 5 BA plates and the resulting 45 plates can be incubated at 38.5 ° C in anaerobic conditions until hemolysis-growth (24 hours) can be observed. This is called pass 2 (P2).

### Step 2: Obtaining the inoculum

Once microscopically checked for the absence of contaminants in growth, the plates can be cut into very small fragments, removing the part of agar in which there is no growth (no visible haemolysis). 2.5 liters of BHI can be prepared and autoclaved. In small amounts of this BHI, the agar fragments can be added from the plates and the mixture can be passed through a mortar until no clots remain. Once all the plates are mixed with the BHI and passed through the mortar, bacteria can be counted with a Neubauer chamber.

The time of the challenge is not particularly limited. The animals might be challenged before, during or after the vaccination, or, where the vaccination comprises several administrations, between the administration of said doses. Preferably the challenge is performed after the vaccination, or, where the vaccination comprises several administrations, after the second and/or after the last administration. Most preferably, the challenge is performed three weeks after the second vaccination and/or five weeks after the first vaccination.

Most preferably, the challenge is performed three weeks after the second vaccination and/or five weeks after the first vaccination with 50 ml of a bacterial suspension comprising 10⁶ bacteria/mL of strain B-204, amounting to a total of 5 x 10⁷ bacteria, administered orally once a day over the course of three days.

### Methods

### Measurement of the rectal temperature

For determination of the rectal temperature, a digital thermometer may be used. The sensor can be placed in the rectum until the number on the display is constant. Evaluation of fever can be established by means of a numerical score, e.g.: 0 (less than 39.5), 1 (between 39.5 and 40.5) and 2 (higher than 40.5). This classification is a modification from that described by Moore *et al.* (1996).

### Culture and isolation of Brachyspira from animals.

According to the present invention, feces samples can be collected individually from animals. Feces samples can be analyzed by culturing samples in CVS selective media and incubating in anaerobic conditions at 41°C. The signal observed can be the haemolysis produced in the culture media and confirmation of the presence of spirochaetes can be made by phase contrast microscopy. A negative result may be given at seventh day of incubation without haemolysis. All cases in which spirochetes are observed may be verified by duplex PCR.

### Duplex PCR to detect Brachyspira hyodysenteriae and Brachyspira pilosicoli.

In accordance with the present invention, a duplex PCR can be used to confirm the presence of *Brachyspira hyodysenteriae* and *Brachyspira pilosicoli* from isolates from swine feces. For this, detection of a DNA fragment of 526 base pairs (bp) of the tlyA gene of B. *hyodysenteriae* and another fragment of 930 bp of the 16S rRNA gene of B. *pilosicoli* may be performed with PCR with specific primers for these fragments. Further teaching can be found In "Råsbäck T, Fellström C, Gunnarsson A, Aspán A. Comparison of culture and biochemical tests with PCR for detection of Brachyspira hyodysenteriae and Brachyspira pilosicoli. J Microbiol Methods 66 (2006): 347 - 353." For example, the sequence from which the fragment of the tlyA gene of B. *hyodysenteriae* is amplified may be the GenBank entry KU215622.1 and the following sequence (SeqID NO: 1):
>KU215622.1 Brachyspira hyodysenteriae strain 49 TlyA (tlyA) gene, partial cds

The following primers may be used for the amplification:
Bh tlyA_F: 5'-GCA GAT CTA AAG CAC AGG AT-3' (SeqID NO: 2)
Bh tlyA_R: 5'-GCC TTT TGA AAC ATC ACC TC-3' (SeqlD NO: 3)

The sequence from which the fragment of the 16S rRNA gene of B. *pilosicoli* is amplified may be the GenBank entry LC259310.1 and the following sequence (SeqID NO: 4):

The following primers may be used for the amplification:
Bp16S_ F: 5'-CAT AAG TAG AGT AGA GGA AAG TTT TT-3' (SeqlD NO: 5)
Bp16S_R: 5'-CTC GAC ATT ACT CGG TAG CAA CAG-3'(SeqID NO: 6)

The template DNA for the PCR may be extracted from a CVS plate or blood agar plate with the isolated strain (visible hemolysis, without defined surface colonies and visible spirochaetes under the microscope). Therefore, a sample may be taken from the hemolytic zone and inserted into a microtube containing 50 µl of dH2O. The sample may then be frozen for 24 hours prior to PCR or boiled for 10-15 minutes. For gram-negative microorganism, these techniques are sufficient to break the cell membrane. After the PCR, the amplified product may be visualized by electrophoresis on an agarose gel and the size of the visible fragments may be used to identify the presence of *Brachyspira hyodysenteriae* and/or *Brachyspira pilosicoli.* Animals that are positive in duplex PCR are declared as excretory animals.

### Indirect Enzyme Linked Immunosorbent Essay (ELISA) for the detection of antibodies against Brachyspira hyodysenteriae

According to the present invention, an Indirect Enzyme Linked Immunosorbent Essay (ELISA) can be used for the detection and quantification of antibodies against *Brachyspira hyodysenteriae.* This assay can be used in accordance with the present invention in a method of quantifying in sera the IgG produced as an immune response against an infection produced by the bacterial strain *Brachyspira hyodysenteriae* or an immunization against an experimental dysentery vaccine. The indirect ELISA uses an immunoenzymatic technique allowing the detection of IgG antibodies against Brachyspira hyodysenteriae in blood sera samples. The test to be used in accordance with the present invention may consist of seven main steps:
1) Coating of the plates: fixation to the solid support (wells) of the whole sonicated bacteria of the vaccine strain H57. Incubation at 4 °C for 18 hours with gentle agitation at 70 rpm.
2) Wash and Blocking step: addition of a carbonate/bicarbonate solution (0.05 M pH 9.6) with bovine serum albumin (1 %) that works as a blocking solution of non-specific binding sites. Incubation at 37 °C for 1 hour with gentle agitation at 70 rpm.
3) Prepare microplate map: a control plate sheet filled in with the identification of the sera that are going to be used as samples put in the exact order that are going to be charged in the ELISA plate, negative control (NC) and positive control (PC), all of them in duplicate, is done for every plate processed or every new run of ELISA.
4) Wash and Sera dilution: Each serum sample is diluted in a mixture of PBS, Tween and bovine serum albumin to 1/1250 concentration and added to a well sensitized with the antigen. The antibodies (Ab) present in the sample bind with the bacterial Ag coating the bottom of the well. The plate is incubated at 37 °C for 1 hour at 70 rpm.
5) Wash and Addition of conjugate: A monoclonal anti- Immunoglobulin G (IgG) conjugated porcine Immunoglobulin (Ig) is added with a peroxidase enzyme. This anti-IgG is fixed on IgG free epitopes that have not bound to Ag bound to the well and form a complex. Incubate at 37 °C for 1 hour at 70 rpm.
6) Wash and Addition substrate: 100 mg of TMB (3,3',5,5'-Tetramethylbenzidin) are dissolved in 10 ml of DMSO (Dimethylsulfoxid) in a citrate/phosphate buffer is used as substrate, and hydrogen peroxide as the reaction catalyst. When the peroxidase enzyme bound to the conjugate recognizes the substrate, it binds to it and the product is transformed by the oxidation action of the hydrogen peroxide into a blue colored product. Incubation takes place for 10 minutes at room temperature in the dark.
7) Stop and Absorbance reading: the reaction is stopped with sulfuric acid and the colorimetric reading of the corresponding optical densities at 450 nm is recorded:
   - In presence of IgG in the serum analyzed an intense yellow colored reaction is observed due to the reaction of the enzyme conjugated to the Anti-IgG Ab which has been bound to the added substrate.
   - In the absence of IgG in the serum analyzed, the anti-IgG Ab has not been bound and has been eliminated in subsequent washes. Therefore there is no colorimetric reaction.
   The optical density at 450nm can therefore be used as a measure for the amount of specific antibodies against *Brachyspira hyodysenteriae.*

### Feces evaluation

Stool quality was measured in the laboratory based on the aspect of samples collected at specific days, using a three score criteria (0, 2, 4) after the 3rd administration (d0) and at days 8, 13 and 37 (d8, d13, d37). Stool scores were 0 for normal or loose consistency, 2 for liquid diarrhea and 4 for mucous or bloody liquid diarrhea, considered severe diarrhea.

### Examples

Unless otherwise defined, all technical and scientific terms used herein have the same meaning as commonly understood by one of ordinary skilled in the art to which this invention belongs. Methods and materials similar or equivalent to those described herein can be used in the practice of the present invention. Additional objects, advantages and features of the invention will become apparent to those skilled in the art upon examination of the description or may be learned by practice of the invention. The following examples and drawings are provided by way of illustration, and they are not intended to be limiting of the present invention.

### Example 1 Culture of the universal vaccine strain

*A Brachyspira hyodysenteriae* strain deposited within the *Collection Nationale de Cultures de Microorganismes* (CNCM), Institut Pasteur, on March 14, 2013, with registration numbers CNCM I-4720 (hereinafter referred to as strain of the present invention) was selected. The strain belongs to the ancestral type of clonal complex II of *Brachyspira hyodysenteriae.*

The isolated bacteria (free from contaminants) were inoculated in agar-blood plates. The plates were kept in anaerobic conditions at 39,5 °C for 4-5 days, until hemolysis areas spread by the agar plates were observed. Agar fragments at the hemolysis borders were inoculated in a new agar-blood plate, and incubated in the same conditions. The bacteria were passed to a new agar-blood plate and, in parallel, to a Fastidious Anaerobe Agar (FAA) plate, and bacteria were cultured in the same conditions for 3-4 days. The bacteria were then transferred to liquid growth medium and cultured.

For fermentation, bacteria were incubated in approx. 4 L of suitable culture medium (such as Brain Heart Infusion media from Merck) at 38,5 °C with light agitation (50 rpm) in an oxygen-free atmosphere. The fermentation occured until the optical density is approx. 1.6 (or until there were around 10⁹ UFC/mL (usually between 15 and 30 hours)). From all tested strains, only the strain CNCM I-4720 was able to grow at an industrial scale.

### Example 2 Inactivation of the bacteria

After fermentation, 4 L (approx.) of culture were pumped into a sterile flask and centrifuged (2 X 7000 rpm, 10 minutes). Resulting pellets were suspended in 1 L sodium acetate buffer 0.1 M. The inactivation of the culture was carried out with an injection of formaldehyde at 0.5 % and incubation during 24 hours at 37 °C with light agitation (50 rpm). On the next day the suspension was centrifuged and resuspended in sterile sodium acetate 0.1 M. The antigen (10^{9 -}10¹⁰ bacteria/mL, approx.) was kept at 4 °C until mixing with adjuvant and excipients for vaccine manufacturing.

### Example 3 Vaccine formula (universal vaccine of the invention)

The vaccine used for the following examples comprises the following components. The term antigen refers to the above-mentioned selected *Brachyspira hyodysenteriae* strain, and the final concentration of antigen is 10⁹ bacterial 2 mL dose for the normal vaccine and 10⁷ bacteria/2 mL dose for the subpotent / reduced dose vaccine.

**Table 1: Composition of the vaccine.**

| (A) Composition of the normal vaccine dose (2L) | | |
|---|---|---|
| **Component** | **Quantity (for 2L dose of vaccine)** | **Percentage/final concentration in the vaccine** |
| Antigen | 200 ml in Sodium Acetate 0,1M | 10⁹ bacteria/2 mL dose |
| Montanide IMS 251 C VG (Seppic) | 400 mL | 20% |
| Sodium acetate 0,1 M (Boente) | 1400 mL | |

### (B) Composition of the reduced vaccine dose for dose confirmation study (2L)

| **Component** | **Quantity (for 2L dose of vaccine)** | **Percentage/final concentration in the vaccine** |
|---|---|---|
| Antigen | 200 ml in Sodium Acetate 0,1M | 10⁷ bacteria/2 mL dose |
| Montanide IMS 251 C VG (Seppic) | 400 mL | 20% |
| Sodium acetate 0,1 M (Boente) | 1400 mL | |

The components were mixed by agitation at 4 °C overnight. For the following experiments, a single dose of the vaccine of the present invention refers to a dose as disclosed in table 1, comprising 2 ml solution with a total of 10⁹ bacteria per dose in the case of the normal dose and 10⁷ bacteria per dose in the case of the reduced dose (the subpotent vaccine).

### Example 4 Pre-regulatory safety study

The study was performed to test of the innocuousness of the Swine dysentery vaccine formulation. Three groups of 8 animals were tested in controlled facilities for the evaluation of regulatory safety parameters (see table 2). The vaccine as described in Example 3 or, in case of group 1, a physiological infusion was administered to the animals.

**Table 2 Animal groups used for the pre-regulatory safety study**

| **GROUP** | **ADMINISTRATION** | **DOSIS** | **TRIAL DAY** | **ANIMALS** |
|---|---|---|---|---|
| **1** | Physiological infusion | 2 ml | D0, D14 | 8 |
| **2** | Vaccine | 2 ml | D0 | 8 |
| **3** | Vaccine | 2 ml | D0, D14 | 8 |

The animals were studied with regard to **abnormal behavior and systemic reactions** such as dyspnoea, mucus, cough, diarrhoea, vomit, paralysis, motile dysfunctions, somnolence, depression and external appearance for at least 14 days after each inoculation. No signs of abnormal behavior and systemic reactions were observed in any animal at any moment of the experiment.

The animals were studied daily with regard to **local reactions at the injection site** until day 14. No local reactions at the injection site were observed in any animal at any moment of the experiment.

The **rectal temperature** was measured at day -1 of first vaccine inoculation, in the administration moment and after 2, 4, 6 and 8 hours post-injection. After that, the temperature was recorded daily 5 days after vaccine injection. The same measurement pattern was carried out for the second inoculations. The results are shown in Fig. 5 and indicate that the injection of the test vaccine in pigs in this trial caused a significant but transitory increase of the temperature of the animals that reverted after 24 hours post inoculation. This fact was observed in both inoculations at day 0 and day 14 of the experiment.

The **weights** of study animals were recorded through a calibrated scale at days 0, 7, 14, 21, 28, 35 and 42. The results are shown in Fig. 6. It can be concluded that the weight development was similar in the different groups.

### Example 5 Inoculation model with Brachyspira hyodysenteriae B 204 from culture plates

The following Standard Operating Procedure was developed in order to obtain an inoculum from blood agar plates of Brachyspira hyodysenteriae strain B204, ATCC 31212 for later use in the development of an infection (i.e. challenge of the animals).

### Step 1: growing the bacteria on a plate

The strain, which was stored at -80 °C, was seeded in 9 blood agar (BA) plates with a 10 µl sowing loop and incubated at 38.5 °C in Anaerobic conditions until hemolysis-growth was observed (1-2 days). This was called pass 1 (P1).

When the strain grew, the absence of contamination was we checked under a microscope. If the strain was pure, growth was collected with a loop, taking agar fragments in depth at the hemolysis edges (which had a better quality of the culture), and deposited on a new BA plate. These fragments were homogenized and spread in zigzag movements along the plate with the loop. Each of the 9 initial plates was seeded into 5 BA plates and the resulting 45 plates were incubated at 38.5 ° C in Anaerobic conditions until hemolysis-growth (24 hours) was observed. This was called pass 2 (P2).

### Step 2: Obtaining the inoculum

Once microscopically checked for the absence of contaminants in growth, the plates were cut into very small fragments, removing the part of agar in which there was no growth (no visible haemolysis). Previously, 2.5 liters of BHI was prepared and autoclaved. In small amounts of this BHI, the agar fragments were added from the plates and the mixture was passed through a mortar until no clots remain. Once all the plates were mixed with the BHI and passed through the mortar, bacteria were counted with a Neubauer chamber, and required to reach a concentration of 10⁶ bacteria/ml.

### Step 3: Inoculation of animals

Each animal to be infected received orally 50 ml of the mixture. To do this, sterile 60 ml syringes were used. The plunger was removed from the syringe and the nozzle was covered with one finger. Then, the syringe body was filled with 50 ml of the mixture. The plunger was then replaced and the remaining air was removed. After doing that, the mixture was introduced directly into the mouth of the animal by expelling the contents of the syringe into the mouth, and checking that the animal takes all the mixture.

### Example 6 Effect of different feeds on the challenge

The objective of the study was to establish an infection model for swine dysentery, disclosing the role of the pre-challenge high protein level in the diet on the clinical outcome. For this purpose, animals were infected with 50 ml of a bacterial culture of B. *hyodysenteriae,* strain B204, with approx.. 5 x10⁶ - 10⁷ bacteria/ml, as described in Example 5.

**Table 3: Groups used in the challenge test**

| **Group** | **Management** |
|---|---|
| Group 1 | 12 pigs orally infected with *B. hyodysenteriae* and fed with hyperproteic feed during 5 days pre-challenge |
| Group 2 | 12 pigs orally infected with *B. hyodysenteriae* with standard feed |
| Group 3 | 12 pigs not infected and fed with hyperproteic feed during 5 days pre-challenge |

As can be seen from Figures 3-6, the challenge was sufficient to infect the challenged groups with diarrhea. In particular the challenge of the pigs fed with hyperproteic feed resulted in animals with a high amount of diarrhea occurrence and reduced body weight compared to the non-infected group.

For determination of the rectal temperature, a digital thermometer was used. The sensor was placed in the rectum until the number on the display was constant. Evaluation of fever was established by means of a numerical score: 0 (less than 39.5), 1 (between 39.5 and 40.5) and 2 (higher than 40.5). This classification is a modification from that described by Moore et al. (1996). Animals scored 2 were considered to have fever. The rectal temperature of the untreated animals is shown in Figure 5.

Animals were weighed at arrival (to perform the randomization while maintaining a weight balance between the three groups), at challenge (D0), and on days D4, D15 and D22. The average body weights are shown in Figure 6.

As a result, it can be concluded that the experimental infection of 10 weeks old pigs by oral route with 50 ml of B. *hyodysenteriae* suspension adjusted to 10⁶ - 10⁷ bacteria/ml per day resulted in a solid model that can be used in the vaccine's efficacy studies. The model was more complete when animals were fed with a hyperproteic diet during five days before the experimental infection.

The clinical signs (diarrhoea, fever, negative effect on body weight), the intestinal lesions, and the shedding of B. *hyodysenteriae* in faeces observed in the animals infected with B. *hyodysenteriae* five days after the ingestion of hyperproteic diet were observed in almost all the animals included in the experimental groups.

### Example 7 Design of the vaccination and challenge experiments

Fifty-six cross-breed healthy pigs were randomly grouped into four pens in order to test the efficacy, safety, safety of the injection site and to justify the dose of the vaccine of the present invention swine dysentery vaccine against B. *hyodysenteriae.* The groups were blind managed by the personnel involved in the clinical monitoring. Figure 7 shows an overview of the challenge protocol used. In general, the challenge protocol with the hyperproteic feed developed in Example 6 was used. In general, animals were treated with the vaccines described in Example 3 or the placebo on days 0 and 14 and their diet was changed to a high protein diet according to Example 6 on day 28. The animals were first vaccinated at an age of 6 weeks. This age ensures that there is no influence of maternal immunity, which can be an issue when testing the vaccine at an earlier age.

**Table 4: Pig groups used in the experiments**

| **Group** | **n** | **Day 0** | **Day 14** | **Challenge** | **Objective** |
|---|---|---|---|---|---|
| Vaccine | 15 | 1^{st} vaccination | 2^{nd} vaccination | D35, 36, 37 | Normal vaccine dose (10⁹ bacteria/dose) for dose confirmation, safety and efficacy studies |
| Placebo | 15 | Placebo | Placebo | D35, 36, 37 | Control for dose confirmation, safety and efficacy studies |
| Subpotent | 15 | 1^{st} vaccination | 2^{nd} vaccination | D35, 36, 37 | Reduced vaccine dose (10⁷ bacteria/dose) for dose confirmation study |

Vaccinated groups were compared with the non-vaccinated or control group in terms of safety (general clinical signs, rectal temperature, body weight control, evaluation of the injection site) and, after challenge with the reference strain B204, in terms of efficacy (onset of immunity) by the evaluation of different parameters. Selected efficacy parameters are aimed to evaluate the disease signs and the production losses in the porcine production (general clinical signs, rectal temperature, diarrhoea evaluation, spirochetes excretion and serology (ELISA test)).

### Example 8 Challenge of vaccinated, vaccinated with a reduced dose and non-vaccinated animals.

The animals from Example 7 that were inoculated received daily doses of 50 mL of a B. *hyodysenteriae* culture of strain B204 at a concentration of 1 x 10⁶ live bacteria/mL during study days 35, 36 and 37 which amounts to about 5 x 10⁷ bacteria/day according to the inoculation protocol described in Example 5. For the administration of the bacterial suspension the animals were first visually identified and then they were fed manually and individually with a 60 mL syringe the stated volume of inoculum. After voluntary ingestion of the required amount of inoculum each animal was marked on the back with a wax crayon. Animals were feed deprived 12 hours before challenge and water deprived 6 hours before challenge during the three challenge days (D35, D36 and D37).

### Example 9 Results of the challenge study

### Indirect Enzyme Linked Immunosorbent Essay (ELISA) for the detection of antibodies against Brachyspira hyodysenteriae.

An Indirect Enzyme Linked Immunosorbent Essay (ELISA) was used for the detection and quantification of antibodies against *Brachyspira hyodysenteriae.* This assay is used to quantify in sera the IgG produced as an immune response against an infection produced by the bacterial strain *Brachyspira hyodysenteriae* or an immunization against an experimental dysentery vaccine. The indirect ELISA uses an immunoenzymatic technique allowing the detection of IgG antibodies against *Brachyspira hyodysenteriae* in blood sera samples. The test consisted of seven main steps:
1) Coating of the plates: fixation to the solid support (wells) of the complete antigen (Ag) of the vaccine strain H57. Incubation at 4 °C for 18 hours with gentle agitation at 70 rpm.
2) Wash and Blocking step: addition of a blocking solution of non-specific binding sites. Incubation at 37 °C for 1 hour with gentle agitation at 70 rpm.
3) Prepare microplate map: a control plate sheet with the samples identification, negative control (NC) and positive control (PC), all of them in duplicate, was done for every plate processed or every new run of ELISA.
4) Wash and sera dilution: Each serum sample was diluted to 1/1250 concentration and added to a well sensitized with the antigen. The antibodies (Ab) present in the sample bind with the bacterial Ag coating the bottom of the well. The plate is incubated at 37 °C for 1 hour at 70 rpm.
5) Wash and addition of conjugate: A monoclonal anti-Immunglobulin G (IgG) conjugated porcine Immunglobulin (Ig) was added with a peroxidase enzyme. This anti-IgG was fixed on IgG free epitopes that have not bound to Ag bound to the well and form a complex. Incubate at 37 °C for 1 hour at 70 rpm.
6) Wash and addition of substrate: TMB/DMSO in a citrate/phosphate buffer was used as substrate, and hydrogen peroxide as the reaction catalyst. When the peroxidase enzyme bound to the conjugate recognizes the substrate, it binds to it and the product is transformed by the oxidation action of the hydrogen peroxide into a blue colored product. Incubation took place for 10 minutes at room temperature in the dark.
7) Stop and absorbance reading: the reaction was stopped with sulfuric acid and the colorimetric reading of the corresponding optical densities at 450 nm is recorded:
   - In presence of IgG in the serum analyzed an intense yellow colored reaction is observed due to the reaction of the enzyme conjugated to the Anti-IgG Ab which has been bound to the added substrate.
   - In the absence of IgG in the serum analyzed, the anti-IgG Ab has not been bound and has been eliminated in subsequent washes. Therefore there is no colorimetric reaction.
   The optical density at 450nm is therefore used as a measure for the amount of specific antibodies against *Brachyspira hyodysenteriae.*

The results from the ELISA experiments demonstrate that the single strain vaccine of Example 3 elicits an immune response based on antibodies. The results from the ELISA experiments also demonstrate that the single strain vaccine of Example 3 elicits a dose-dependent immune response. The results of the ELISA experiments are shown in Figure 11.

### Culture and isolation of strains from faeces

Aliquots of approximately 5 g of faeces were collected individually in sterile plastic flasks from all Study groups (A, B, C and D). Between 5-10gr of faeces were collected from all animals from groups A, B and D at days D35, D41, D43, D45, D48, D50, D52, D56, D59, D64 and D70. Faeces was collected directly from the animals by inducing defecation by palpation if necessary. All faecal samples were cultured in CVS selective media and incubated in anaerobic conditions at 41°C. The signal observed was the haemolysis produced in the culture media and confirmation of the presence of spirochaetes was made by phase contrast microscopy. A negative result was given at seventh day of incubation without haemolysis. All cases in which spirochetes were observed were verified by duplex PCR.

### Duplex PCR to detect Brachyspira hyodysenteriae and Brachyspira pilosicoli.

Duplex PCRs were used to confirm the presence of *Brachyspira hyodysenteriae* and *Brachyspira pilosicoli* from isolates from swine feces. For this, a DNA fragment of 526 base pairs (bp) of the tlyA gene of B. *hyodysenteriae* and another fragment of 930 bp of the 16S rRNA gene of B. *pilosicoli* was detected with a PCR with specific primers for these fragments. The sequence from which the fragment of the tlyA gene of B. *hyodysenteriae* was amplified has the GenBank entry KU215622.1 and the following sequence (SeqlD NO: 1):
>KU215622.1 Brachyspira hyodysenteriae strain 49 TlyA (tlyA) gene, partial cds

For the PCR, the following primers were used:
Bh tlyA_F: 5'-GCA GAT CTA AAG CAC AGG AT-3' (SeqID NO: 2)
Bh tlyA_R: 5'-GCC TTT TGA AAC ATC ACC TC-3' (SeqlD NO: 3)

The sequence from which the fragment of the 16S rRNA gene of B. *pilosicoli* was amplified has the GenBank entry LC259310.1 and the following sequence (SeqID NO: 4):

For the PCR, the following primers were used:
Bp16S_ F: 5'-CAT AAG TAG AGT AGA GGA AAG TTT TT-3' (SeqlD NO: 5)
Bp16S_R: 5'-CTC GAC ATT ACT CGG TAG CAA CAG-3'(SeqID NO: 6)

The template DNA for the PCR was extracted from a CVS plate or blood agar plate with the isolated strain (visible hemolysis, without defined surface colonies and visible spirochaetes under the microscope). A sample was taken from the hemolytic zone and inserted into a microtube containing 50 µl of dH2O. The sample was then frozen for 24 hours prior to PCR or boiled for 10-15 minutes. For gram-negative microorganism, these techniques are sufficient to break the cell membrane. After the PCR, the amplified product was visualized by electrophoresis on an agarose gel and the size of the visible fragments was used to identify the presence of *Brachyspira hyodysenteriae* and/or *Brachyspira pilosicoli.* Animals that were positive in duplex PCR were declared as excretory animals.

The results are presented in figure 10 which shows the percentage of animals shedding B. *hyodysenteria* at different times after the challenge. Shedding (an indication of infection by the challenging strain) is comparable at the beginning of the observation (which indicates that all the groups are equally infected), but it is significantly reduced at the middle and at the end of the observation time in the optimal dose vaccinated group. This is consistent in the reduction of the clinical signs (as measured by the consistency of the stools as indicative of moderate or severe diarrhea) and shows that the vaccine helps the immune system to get rid of the bacteria. This shows that the vaccine of the present invention is effective in controlling infection with *B. hyodysenteria* and furthermore is able to prevent spreading to other individuals.

### Faeces and diarrhoea evaluation

Stool quality was evaluated in the laboratory on samples collected at different days after the start of the diet change. The classification used in this analysis was
0: normal or loose faeces.
2: liquid diarrhoea
4: Presence of mucus and blood in faeces (typical aspect and smell).

Diarrhoea was evaluated in animals from vaccine, placebo and subpotent vaccine groups.

The results are presented in Figures 8 and 9, showing the average diarrhoea score index and the percentage of animals with severe diarrhoea in the different groups at different times after the challenge. The figures clearly show that both the average diarrhoea score index as well as the percentage of animals with severe diarrhoea is dramatically lower in animals that have been treated with the potent vaccination of the present invention compared to both the animals treated with a subpotent vaccine or a placebo solution. Treatment with a subpotent vaccine still shows a reduction of both the diarrhoea score index and the fraction of animals with severe diarrhea compared to the animals treated with a placebo solution. This demonstrates that the vaccine of the present invention can be used to prevent and/or treat swine dysentery and remarkably is also effective against a heterologous *B. hyodysenteria* strain.

In general, the vaccinated animals show less diarrhoea, when they develop diarrhoea develop it at later stages, and resolve diarrhoea sooner than non-vaccinated animals or animals vaccinated with a low dose vaccine.

## Claims

1. A composition for use in the prevention and/or treatment of swine dysentery, wherein the swine dysentery is caused by *Brachyspira hyodysenteriae* which is of another clonal complex and/or another MLVA type than the strain CNCM 1-4720, said composition comprising a single strain of *Brachyspira hyodysenteriae,* wherein no other *Brachyspira hyodysenteriae* strain is present in the composition, and wherein said strain is the strain with the deposit number CNCM I-4720, wherein the bacteria are inactivated.

2. The composition for use according to claim 1, wherein the occurrence of mucous and/or bloody diarrhea is reduced.

3. The composition for use according to claim 1, wherein the appearance of clinical signs is delayed and/or overall severity of diarrhea is reduced.

4. The composition for use according to claim 1, wherein shedding of bacteria is prevented and/or the time thereof is reduced.

5. The composition for use according to any of claims 1 to 4, wherein the bacteria are present in a concentration of at least between 10⁸ and 10⁹ of total bacteria/mL, of between 10⁸ and 10⁹ of total bacteria/mL, or in a concentration of 5 * 10⁸ total bacteria/ml, and/or wherein the administered dosage is between 1 mL to 5 mL, optionally 2 mL, and/or wherein the bacteria are present in an amount of between 10⁷ and 10¹¹ of total bacteria/dose, between 10⁸ and 10¹⁰ of total bacteria/dose, or in an amount of 10⁹ of total bacteria/dose.

6. The composition for use according to any of claims 1 to 5, wherein the composition is administered by parenteral administration, preferably by intramuscular administration.

7. The composition for use according to any of claims 1 to 6, wherein the prevention or treatment is a prevention or treatment of a pig, preferably of a domestic pig.

## Patentansprüche

1. Zusammensetzung zur Verwendung bei der Prävention und/oder Behandlung von Schweinedysenterie, wobei die Schweinedysenterie durch *Brachyspira hyodysenteriae* veranlasst wird, die aus einem anderen klonalen Komplex und/oder einen anderen MLVA-Typ als den Stamm CNCM 1-4720 besteht, wobei die Zusammensetzung einen Einzelstamm von *Brachyspira hyodysenteriae* umfasst, wobei kein anderer Stamm von *Brachyspira hyodysenteriae* in der Zusammensetzung vorhanden ist, und wobei der Stamm der Stamm mit der Hinterlegungsnummer CNCM 1-4720 ist, wobei die Bakterien inaktiviert sind.

2. Zusammensetzung zur Verwendung nach Anspruch 1, wobei das Auftreten von Schleim und/oder blutigem Durchfall reduziert wird.

3. Zusammensetzung zur Verwendung nach Anspruch 1, wobei das Erscheinen klinischer Anzeichen verzögert und/oder der Gesamt-Schweregrad des Durchfalls reduziert wird.

4. Zusammensetzung zur Verwendung nach Anspruch 1, wobei das Ausscheiden von Bakterien verhindert und/oder deren Zeit verkürzt wird.

5. Zusammensetzung zur Verwendung nach einem der Ansprüche 1 bis 4, wobei die Bakterien in einer Konzentration von mindestens zwischen 10⁸ und 10⁹ Gesamtzahl an Bakterien/ml, von zwischen 10⁸ und 10⁹ Gesamtzahl an Bakterien/ml oder in einer Konzentration von 5 * 10⁸ Gesamtzahl an Bakterien/ml vorhanden sind, und/oder
wobei die verabreichte Dosierung zwischen 1 ml und 5 ml, wahlweise 2 ml ist, und/oder wobei die Bakterien in einer Menge von zwischen 10⁷ und 10¹¹ Gesamtzahl an Bakterien/Dosis, zwischen 10⁸ und 10¹⁰ Gesamtzahl an Bakterien/Dosis oder in einer Menge von 10⁹ Gesamtzahl an Bakterien/Dosis vorhanden sind.

6. Zusammensetzung zur Verwendung nach einem der Ansprüche 1 bis 5, wobei die Zusammensetzung durch parenterale Verabreichung, bevorzugt durch intramuskuläre Verabreichung, verabreicht wird.

7. Zusammensetzung zur Verwendung nach einem der Ansprüche 1 bis 6, wobei die Prävention oder Behandlung eine Prävention oder Behandlung eines Schweins, bevorzugt eines Hausschweines, ist.

## Revendications

1. Composition destinée à être utilisée dans la prévention et/ou le traitement de la dysenterie porcine, dans laquelle la dysenterie porcine est causée par *Brachyspira hyodysenteriae* qui est d'un autre complexe clonal et/ou un autre type de MLVA que la souche CNCM I-4720, ladite composition comprenant une souche unique de *Brachyspira hyodysenteriae,* dans laquelle aucune autre souche de *Brachyspira hyodysenteriae* n'est présente dans la composition, et dans laquelle ladite souche est la souche portant le numéro de dépôt CNCM I-4720, dans laquelle les bactéries sont inactivées.

2. Composition pour une utilisation selon la revendication 1, dans laquelle l'occurrence de diarrhée muqueuse et/ou sanguinolente est réduite.

3. Composition pour une utilisation selon la revendication 1, dans laquelle l'apparition de signes cliniques est retardée et/ou la sévérité globale de la diarrhée est réduite.

4. Composition pour une utilisation selon la revendication 1, dans laquelle la dissémination de bactéries est empêchée et/ou son temps est réduit.

5. Composition pour une utilisation selon l'une quelconque des revendications 1 à 4, dans laquelle les bactéries sont présentes en une concentration au moins comprise entre 10⁸ et 10⁹ de bactéries au total/ml, entre 10⁸ et 10⁹ de bactéries au total/ml, ou en une concentration de 5 * 10⁸ bactéries au total/ml, et/ou
dans laquelle le dosage administré est compris entre 1 ml et 5 ml, facultativement 2 ml, et/ou
dans laquelle les bactéries sont présentes en une quantité comprise entre 10⁷ et 10¹¹ de bactéries au total/dose, entre 10⁸ et 10¹⁰ de bactéries au total/dose, ou en une quantité de 10⁹ de bactéries au total/dose.

6. Composition pour une utilisation selon l'une quelconque des revendications 1 à 5, dans laquelle la composition est administrée par administration parentérale, préférablement par administration intramusculaire.

7. Composition pour une utilisation selon l'une quelconque des revendications 1 à 6, dans laquelle la prévention ou le traitement est une prévention ou un traitement d'un porc, préférablement d'un porc domestique.


### Sequence Listing

### SEQUENCE LISTING

<110> Aquilon CYL S.L
<120> Single strain dysentery vaccine
<130> to be determined
<160> 6
<170> BiSSAP 1.3.6
<210> 1
   <211> 775
   <212> DNA
   <213> Brachyspira hyodysenteriae
<220>
   <223> TlyA (tlyA) gene, partial cds, GenBank: KU215622.1
<400> 1
   gtaaatatga gagataaaga aagaaattct ctttctataa taaaatcttt ccttggatta 60
   taatactaat ataaatgcga ttagatgaat atgtgcatag tgaaggctat acagaaagca 120
   gatctaaagc acaggatata atactagccg gttgtgtttt tgttaatgga gtaaaggtaa 180
   cttctaaggc tcataaaata aaagatactg ataatataga agttgttcag aatataaaat 240
   atgtatcaag agctggagaa aaattagaaa aggcgtttgt agaatttgga atatctgtag 300
   aaaataaaat atgtttagat ataggagctt ctacaggagg atttacagat tgtctgctta 360
   agcatggtgc taaaaaagtt tatgctcttg atgtaggaca taatcagcta gtttataaac 420
   ttcgtaatga taatagggta gtgtcaatag aagatttcaa tgccaaagat ataaataaag 480
   aaatgttcaa tgatgaaatc ccatctgtaa tagtaagtga cgtatcattt atatcaataa 540
   caaaaatagc accaatcata tttaaagaat taaataattt agagttttgg gtaactttaa 600
   taaaaccaca atttgaagct gaaagaggtg atgtttcaaa aggcggtata atacgagatg 660
   atatacttag agaaaaaata ttaaataatg ctatttcaaa gataatagac tgcggattta 720
   aagaagttaa tagaaccatc tctcctataa aaggtgctaa aggtaatata gaata 775
<210> 2
   <211> 20
   <212> DNA
   <213> Artificial Sequence
<220>
   <223> Bh tlyA_F primer
<400> 2
   gcagatctaa agcacaggat 20
<210> 3
   <211> 20
   <212> DNA
   <213> Artificial Sequence
<220>
   <223> Bh tlyA_R primer
<400> 3
   gccttttgaa acatcacctc 20
<210> 4
   <211> 1346
   <212> DNA
   <213> Brachyspira pilosicoli
<220>
   <223> gene for 16S ribosomal RNA, partial sequence
<400> 4
   atgcagtcga gcgggcttat tcgggcaact ggataagtta gcggcgaact ggtgagtaac 60
   acgtaggtaa tctgccgtga agtgggggat aacccatgga aacatggact aataccgcat 120
   atactcttgc tacataagta gagtagagga aagttttttc gcttcacgat gagcctgcgg 180
   cctattagcc tgttggtagg gtaatggcct accaaagcta cgataggtag ccgacctgag 240
   agggtgaccg gccacattgg gactgagata cggcccagac tcctacggga ggcagcagct 300
   gagaatcttc cacaatggac gaaagtctga tggagcgaca tcgcgtgagg gatgaaggcc 360
   ttcgggttgt aaacctcgga aattatcgaa gaatgagtga cagtagataa tgtaagcctc 420
   ggctaactac gtgccagcag ccgcggtaat acgtaggagg caaacgttgc tcggatttac 480
   tgggcgtaaa gggtgagtag gcggatttat aagtctaagg tgaaagaccg aagctcaact 540
   tcgggaacgc ctcggatact gtaagtcttg gatattgtag gggatgatgg aattctcggt 600
   gtagcggtgg aatgcgcaga tatcgagagg aacacctata gcgaaggcag tcatctgggc 660
   atttatcgac gctgaatcac gaaagctagg ggagcaaaca ggcttagata ccctggtagt 720
   cctagccgta aacgttgtac actaggtgct tctatttaaa taggagtgcc gtagctaacg 780
   tcttaagtgt accgcctgag gagtatgccc gcaagggtga aactcaaaga aattgacggg 840
   tccccgcaca agtggtggag catgtggttt aattcgatga tacgcgaaaa accttacctg 900
   ggtttgaatt gttagatgaa tgatttagag ataagtcaga ccgcaaggac gtttaacata 960
   ggtgctgcat ggctgtcgtc agctcgtgtc gtgagatgtt gggttaagtc ccgcaacgag 1020
   cgcaaccctc accctctgtt gctaacgagt aatgtcgagc actcttaggg gactgcctac 1080
   gttcaagtag gaggaaggtg gggatgatgt caagtcctca tggcccttat gtccagggct 1140
   acacacgtgc tacaatggca agtacaaaga gaagcaagac cgcgaggtgg agcaaaactc 1200
   aaaaaagttg cctcagttcg gattggagtc tgaaactcga ctccatgaag ttggaatcac 1260
   tagtaatcgt agatcagaac gctacggtga atacgttccc ggggattgta cacaccgccc 1320
   gtcacgccat cggagttggt tttacc 1346
<210> 5
   <211> 26
   <212> DNA
   <213> Artificial Sequence
<220>
   <223> Bp16S_ F: 5 primer
<400> 5
   cataagtaga gtagaggaaa gttttt 26
<210> 6
   <211> 24
   <212> DNA
   <213> Artificial Sequence
<220>
   <223> Bp16S_R: 5 primer
<400> 6
   ctcgacatta ctcggtagca acag 24